(19) Europäisches Patentamt
European Patent Office
Office européen des brevets

(11) Publication number: **0 393 227 B1**

(12) **EUROPEAN PATENT SPECIFICATION**

(45) Date of publication of patent specification: **08.03.95**

(51) Int. Cl.[6]: **C07H 15/04**, C07H 13/04, G01N 33/569, G01N 33/577, A61K 39/118, C12N 15/52, C12P 19/26

(21) Application number: **89107260.5**

(22) Date of filing: **21.04.89**

# (54) Tetrasaccharide derivatives, methods for their manufacture and their use.

(43) Date of publication of application:
**24.10.90 Bulletin 90/43**

(45) Publication of the grant of the patent:
**08.03.95 Bulletin 95/10**

(84) Designated Contracting States:
**AT BE CH DE FR GB IT LI LU NL SE**

(56) References cited:
**EP-A- 0 253 912**

**CARBOHYDRATE RESEARCH, vol. 183, 1988, pages 183-199, Elsevier SciencePublishers B.V., Amsterdam, NL; P. KOSMA et al.: "Synthesis of a trisaccharideof 3-deoxy-d-manno-2-octulopyranosylonic acid (KDO) residues related to thegenusspecific lipopolysaccharide epitope of Chlamydia"**

**CARBOHYDRATE RESEARCH, vol. 180, 1988, pages 19-28, Elsevier Science PublishersB.V., Amsterdam, NL; P. KOSMA et al.: "Synthesis of polyacrylamide copolymers**

(73) Proprietor: **Brade, Helmut, Dr. med.**
**Seekoppel 16**
**D-23829 Kükels (DE)**

(72) Inventor: **Brade, Helmut, Dr. med.**
**Seekoppel 16**
**D-2361 Kükels (DE)**
Inventor: **Kosma, Paul, Dr.**
**Institut für Chem. der Universität für Bodenkultur**
**Gregor-Mendel-Strasse 33**
**A-1180 Wien (AT)**

(74) Representative: **TER MEER - MÜLLER - STEINMEISTER & PARTNER**
**Mauerkircherstrasse 45**
**D-81679 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 393 227 B1

containing alfa-(2-4)-beta-, beta-(2-4)-beta-, and beta-(2-4)-alfa-linkedomicron-(3-deoxy-d-manno-2-octulopyranosylonate)-(3-deoxy--d-manno-2-octulopyranosylono) (KDO) residues"

TETRAHEDRON LETTERS, vol. 27, no. 10, 1986, pages 1135-1138, Pergamon PressLtd, GB; H. PAULSEN et al.: "Synthese eines 3-desoxy-d-manno-2-Octulosonsäure(KDO) -haltigen Tetrasaccharides und dessen Strukturvergleich mit einemAbbauprodukt aus Bakterien-lipopolysacchariden"

## Description

FIELD OF THE INVENTION

This invention relates to tetrasaccharide derivatives, methods for their manufacture and their use as reagents or compositions for the diagnosis and therapy of human and animal chlamydial infections.

BACKGROUND OF THE INVENTION

Chlamydiae are pathogenic, obligatory intracellular parasites causing a variety of diseases in animals and humans.

There are two species, Chlamydia trachomatis and Chlamydia psittaci. Chlamydia trachomatis is a specific human pathogen, whereas Chlamydia psittaci is predominantly a pathogen of a variety of non-primate species. Diseases caused by Chlamydia trachomatis present a major health problem throughout the world. In developing countries, Chlamydia trachomatis causes hyperendemic trachoma which is the world's leading cause of preventable blindness. In industrialized nations, Chlamydia trachomatis is a sexually transmitted pathogen that causes an array of genital tract and associated infections whose incidence is increasing at epidemic rates. Chlamydia-like organisms have also been identified in tissues of spiders (Coelotus lucuosus) and clams (Mercenarie merchenaria), suggesting that chlamydiae are ubiquitous parasites present throughout the animal kingdom. Samples of chlamydiae, such as those referred to above are commercially available from depositories, such as The American Type Culture Collection.

Chlamydiae have a unique developmental cycle including metabolically active, non-infectious, multiplying reticulate bodies and metabolically inactive but infectious elementary bodies. Surface components of these unique bacteria have been assumed to participate in the early steps during infection (adhesion and penetration) and may be responsible for the inhibition of phagosome-lysosome fusion, a characteristic feature of the chlamydial infection.

These surface components of Chlamydiae also represent antigens among which genus-, species-, and subspecies antigens have been found, giving rise to the induction of specific antibodies during infection. Although there is a considerable knowledge on the serological and biological properties of these antigens little is known on the chemistry of these structures. One of the major antigens is a heat-stable, genus-specific glycolipid antigen which has been proposed to be similar to the lipopolysaccharide (LPS) of gram-negative bacteria. Recently, it has been shown definitely, by chemical means, that the glycolipids of Chlamydia trachomatis serotype L2 (Nurminen, M., E. Th. Rietschel, and H. Brade, 1985. Infect. Immun. 48: 573-575) and of Chlamydia psittaci strain PK 5082 (Brade L., S. Schramek U. Schade and H. Brade, 1986. Infect. Immun., 54:568-574) are in fact typical lipopolysaccharides since they contain characteristic structural elements such as D-glucosamine, 3-deoxy-D-manno-2-octulosonic acid (KDO), and 3-hydroxy long chain fatty acids, components which have been detected in all lipopolysaccharides investigated so far. Further evidence for the similarity between chlamydial and enterobacterial lipopolysaccharide was obtained from serological investigations showing a cross-reaction of chlamydial with enterobacterial lipopolysaccharide of the Re-chemotype and with lipopolysaccharide from Acinetobacter calcoaceticus.

EP-A-0 253 912 discloses oligosaccharides that comprise the genus-specific epitope of chlamydial lipopolysaccharide and which can be used as specific inhibitors of antigen-antibody reactions. These oligosaccharides are disaccharides and trisaccharides of 3-deoxy-D-manno-octulopyranosylonic acid (KDO). These oligosaccharides are disclosed to be useful also as immunogens and as antigens in the diagnosis and therapy of human and animal chlamydial infections.

It has furtheron been shown that the genus-specific glycolipid antigen of chlamydia contains a KDO-trisaccharide of the sequence α-KDOp-2.8-α-KDOp-2.4-α-KDOp as the immunodominant group (H. Brade, L. Brade and F. E. Nano, Proc. Natl. Acad. Sci. (USA) 84: 2508-2512 and: P. Kosma, G. Schulz, and H. Brade, Carbohydr. Res. 183:183-199 (1988)).

However, all the reagents related to the diagnosis of chlamydial infections (antigens or antibodies) are based upon the understanding that all chlamydiae have a common LPS antigen which gives rise to the production of antibodies during an infection. This is the major antigen of chlamydiae and is used in diagnostic methods as a marker for the detection of chlamydiae in specific human or animal material making use of specific antibodies against chlamydiae-specific LPS structures. Up to now these properties have been described and used in a phenomenological way without having knowledge of the exact chemical structure of the chlamydiae-specific determinants or the epitope specificity of the used antibodies. According to present knowledge chlamydiae-specificity is defined by monoclonal antibodies. which react with all chlamydiae and only with chlamydiae. A prototype of such an antibody is the known monoclonal

antibody L2I-6 which has been deposited with the American Type Culture Collection (ATCC). As has been demonstrated by the inventor by chemical, immunochemical and serological determinations a trisaccharide of KDO is a necessary component of a chlamydia specific antigen (see EP-A-0 253 912). However, it was not known up to now as to what are the necessary criteria for the chlamydiae-specificity. The object of the invention therefore is the provision of a chemically characterized oligosaccharide having chlamydiae-specificity.

It has now been found that the chemical structure having this chlamydiae-specificity comprises the tetrasaccharide derivatives of the following general formula (I)

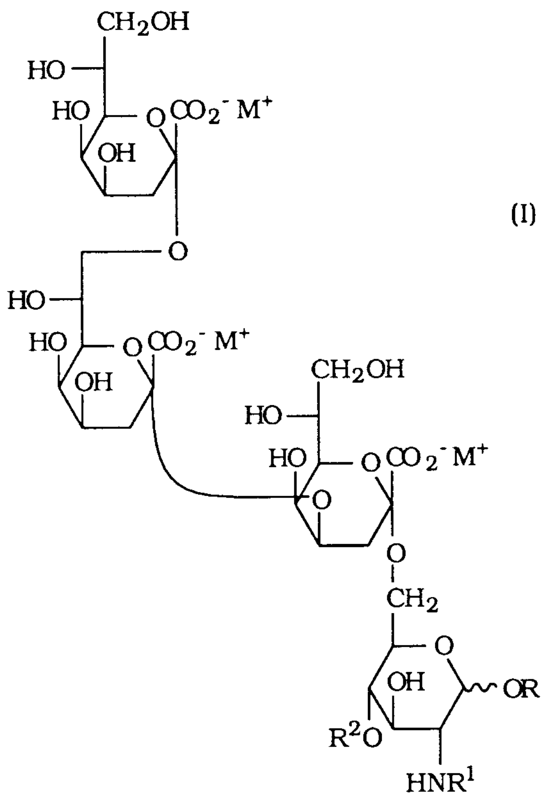

(I)

wherein
R is hydrogen, alkyl, alkenyl, aminoalkyl or the residue of an aldose, a ketose or a sugar alcohol carrying phosphate, sulfate, amino or carboxyl groups and having up to 4 to 8 carbon atoms,
$R^1$ is hydrogen or an acyl group,
$R^2$ is hydrogen or $PO(OH)_2$ and
$M^+$ is a neutralizing cation.

The subject matter of the present invention therefore are the above tetrasaccharide derivatives, methods for their manufacture and their use for the preparation of reagents and compositions for the diagnosis and therapy of human and animal chlamydial infections.

It could be shown that these tetrasaccharide derivatives having the above chemical structure are chlamydiae-specific as demonstrated against the monoclonal antibodies L2I-6 or specific monoclonal antibodies made and used as shown below.

A further subject matter of the present invention are methods for the manufacture of these tetrasaccharide derivatives by chemical synthesis or by real extraction from chlamydiae or recombinant gram-negative bacteria transformed with the plasmid pFEN207, which has been deposited with the American Type Culture Collection (ATCC) in Eschericia coli GM109 (pFEN207) which has obtained the deposition number 53041.

A further subject matter of the invention is the use of these tetrasaccharide derivatives as antigens for the direct assay of lipopolysaccharide antibodies against chlamydiae, as inhibitors of the chlamydiae-specific antibody reaction as immunogens for the manufacture of monclonal or polyclonal antibodies, as active agents for the manufacture of affinity media for preparative purification of monoclonal or polyclonal

antibodies, as active vaccines, as reagents for the characterization of the epitope specificity of monoclonal or polyclonal antibodies, as therapeutic agents for the blocking of lipopolysaccharide-recognizing receptor structures on host cells or as ligands for coupling with carrier materials.

DETAILED DESCRIPTION OF THE PREFERRED EMBODIMENTS

The tetrasaccharide derivatives of the invention correspond to the following general formula (I)

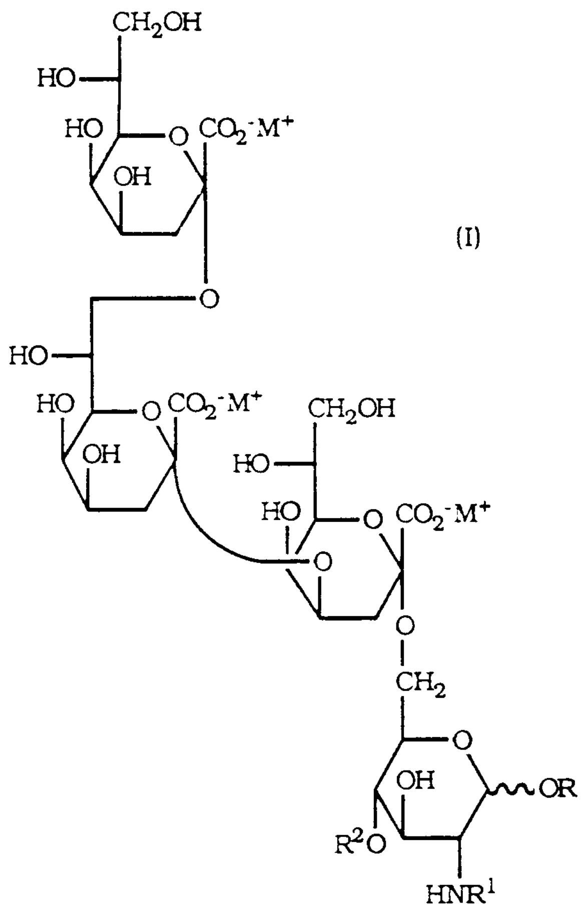

(I)

wherein
R is alkyl, preferably alkyl having 1 to 4 carbon atoms, such as methyl, ethyl, propyl, isopropyl, butyl, isobutyl or tert.-butyl, alkenyl, preferably alkenyl having 3 to 8 carbon atoms, aminoalkyl wherein the alkyl residue preferably has 1 to 4 carbon atoms, or a residue of a sugar, a sugar alcohol or a sugar derivative, such as glucosaminitol or glucosamine, preferably a sugar residue such as a residue of aldose or ketose, a sugar alcohol or a residue of a sugar derivative such as sugars carrying phosphate, sulfate, amino or carboxyl groups and having up to 4 to 8 carbon atoms preferentially hexoses, hexitols or hexose derivatives, respectively.
$R^1$ is hydrogen or an acyl group having 2 to 24 carbon atoms, preferably 1-4 carbon atoms or 3-hydroxyacyl groups having 4 to 24 carbon atoms,
$R^2$ is hydrogen or $PO(OH)^2$ and
$M^+$ is a neutralizing cation, preferably a cation of sodium, potassium, calcium, ammonium or substituted ammonium, such as trialkyl ammonium like triethyl ammonium.

A specifically preferred tetrasaccharide derivative of the invention is 3-deoxy-α-D-manno-2-octulopyranosyl-onate-(2 → 8)-3-deoxy-α-D-manno-2-octulopyranosyl-onate-(2 → 4)-3-deoxy-α-D-manno-2-octulopyranosyl-onate-(2 → 6)-2-amino-2-deoxy-β-D-glucopyranosyl-(1 → 6)-2-amino-2-deoxy-D-glucitol having the following formula (II).

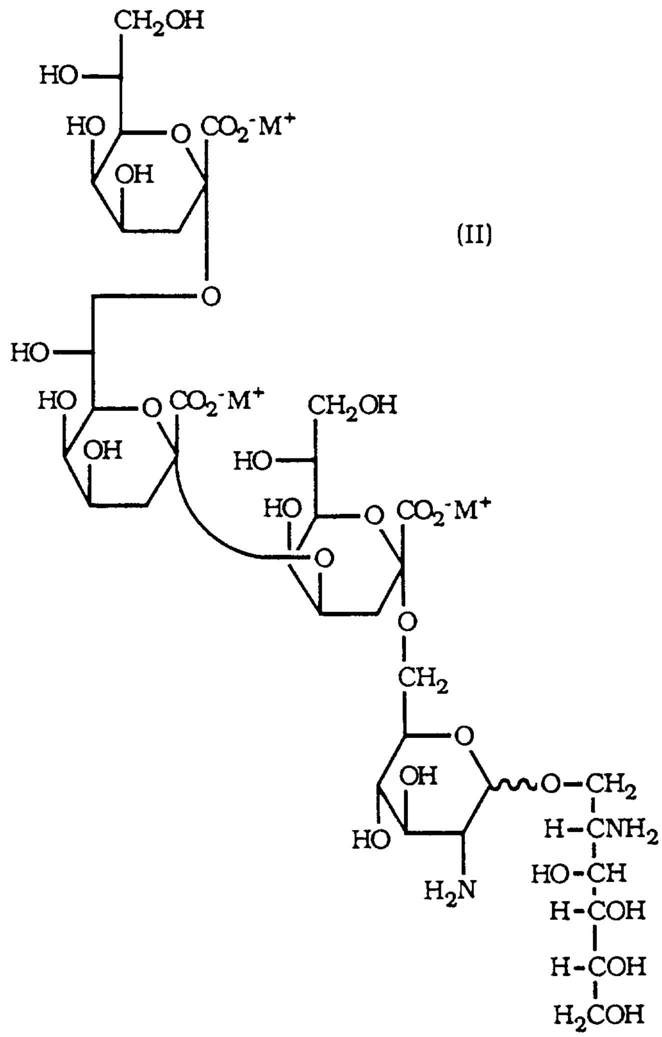

(II)

The process of the present invention for the manufacture of the tetrasaccharide derivatives of the above general formulae (I) and (II), according to chemical synthesis comprises reacting a disaccharide of the general formula (III)

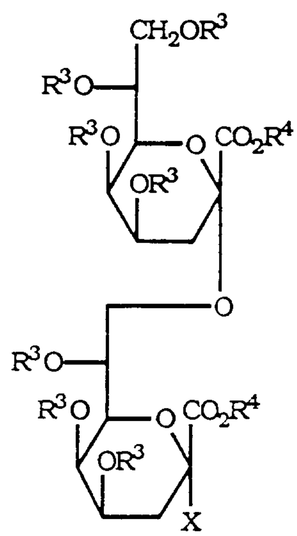

(III)

wherein $R^3$ are protecting groups preferably acyl groups having 2 to 4 carbon atoms, more preferably acetyl groups,
$R^4$ are alkyl groups, preferably methyl groups and
X is halogen, preferably a chlorine or bromine, by a method known per se with a disaccharide of the general formula (IV) or a trisaccharide of the general formula (V)

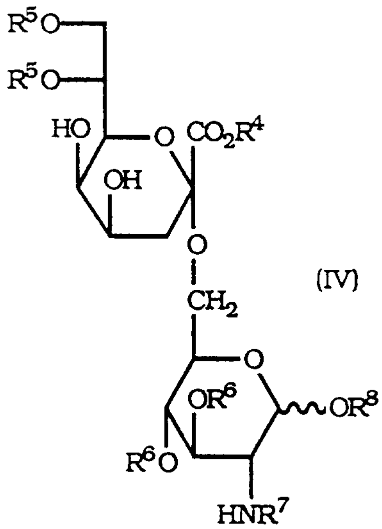
(IV)

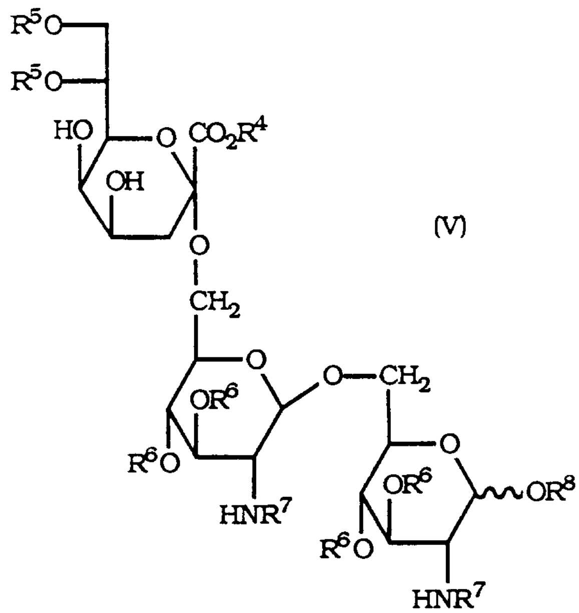
(V)

wherein $R^4$ is as defined above,
$R^5$ and $R^6$ independently are bifunctional protecting groups, such as cyclic acetal or ketal groups, ester functions or silyl ether groups,
$R^7$ independently are acyl groups having 2 to 24 carbon atoms or 3-hydroxyacyl groups having 4 to 24 carbon atoms and
$R^8$ are alkyl groups having 1 to 4 carbon atoms or alkenyl groups having 3 to 8 carbon atoms,
in an inert solvent and in the presence of a heavy metal salt catalyst, chromatographically separating the tetrasaccharide derivative and thereafter eliminating the protecting groups by a method known per se.

In the above general formulae (III), (IV) and (V) the alkyl groups preferably have 1 to 4, more preferably 2 to 4 carbon atoms, the halogens preferably comprise fluorine, chlorine and bromine, the alkenyl groups preferably comprise 3 to 8 carbon atoms, the protecting groups $R^3$ preferably are acyl groups having 2 to 4 carbon atoms, more preferably acetyl groups, $R^4$ preferably are methyl groups, the protecting groups $R^5$ and $R^6$ are bifunctional protecting groups, such as cyclic acetal or ketal groups, ester functions or silyl esters, whereas groups $R^7$ are are acyl groups having 2 to 24 carbon atoms or 3-hydroxyacyl groups preferentially having 4 to 24 carbon atoms.

The preferred inert solvents used in these reactions are highly polar aprotic solvents, such as acetonitril, dimethylsufoxide, hydrocarbons, halogenated hydrocarbons, such as dichloromethane, or ethers, such as tetrahydrofurane.

As heavy metal salt catalysts those of mercury, silver or thallium are used, such as mercury cyanide or mercury bromide.

The starting compounds of the general formulae (III), (IV) and (V) are known or can easily be obtained by the man of the art making use of general synthesis methods.

It is furtheron possible to manufacture the tetrasaccharide derivative of the invention by

a) transforming the plasmide pFEN 207 in a RE-chemotype of a gram-negative bacterium (hereinafter designated as recombinant RE-bacteriae - rRE),

b) culturing said recombinant RE-bacteriae by a method known per se (Nano, F. E. and H. D. Caldwell. Science 228:742-744 (1985)),

c) isolating and purifying the lipopolysaccharide (rRE-LPS) of the multiplied recombinant RE-bacteriae by a method known per se,

(d) dissolving rRE-LPS in anhydrous hydrazine (preferably having a concentration of 1 to 20 mg/ml) stirring for preferably 30 min under a nitrogen atmosphere at a temperature of 37 ° C, purifying the product, preferably by precipitating by the addition of acetone, separating, purifying and drying to obtain the product (rRE-LPS-$N_2H_4$-37).

(e) suspending and stirring the above product rRE-LPS-$N_2H_4$-37 at a temperature of 0 ° C for preferably 48 h in aqueous HF (preferably 48% aqueous HF), purifying, neutralizing and drying the product which by a preferred embodiment is carried out by precipitating a material by adding ethanol, separating, purifying, drying, dissolving the product in water (1 to 20 mg/ml) neutralizing with NaOH or triethylamine and lyophilizing the product (rRE-LPS-$N_2H_4$-37-HF),

(f) dissolving the above product rRE-LPS-$N_2H_4$-37-HF in water (preferably at a concentration of 1 to 20 mg/ml), reducing the material, preferably by adding sodium borohydride, at room temperature for preferably 15 min to 2 h, dialyzing and drying the product rRE-LPS-$N_2H_4$-37-HFred which is preferably carried out by adding acetic acid, dialyzing against water and lyophilizing the product, and

(g) adding hydrazine to the above product rRE-LPS-$N_2H_4$-37-HFred, stirring under a nitrogen atmosphere at an elevated temperature, purifying, drying and isolating the tetrasaccharide derivatives obtained by preparative high voltage electrophoresis, wherein this last step g is preferably carried out by adding 0.1 to 2 ml/20 mg anhydrous hydrazine to the material dried in the vacuum with a drying agent, stirring for 5 to 14 days under nitrogen atmosphere at a temperature of 85 to 100 ° C, precipitating by adding acetone, separating, purifying, drying, dissolving in water (5 to 100 mg/ml) and isolating the tetrasaccharide derivatives by preparative high voltage electrophoresis at a pH of 2.8 and 50 V/cm, and

(h) introducing the desired substituents R by methods known per se.

As has been described above, by making use of the plasmid pFEN 207 recombinant mutants of different types of bacteria can be obtained, which synthesize a LPS having a serological chlamydia-specificity. It is also possible to obtain the above tetrasaccharide derivatives by not making use of the above disclosed recombinant technology, but by starting from chlamydiae and isolating and purifying chlamydiae LPS making use of the above stated separating and reaction steps.

The tetrasaccharide derivatives of the present invention are very useful and chemically fully characterized products which can be used

1. as antigens for the direct determination of LPS-antibodies against chlamydiae;
2. as inhibitors for the chlamydia-specific antigen/antibody reaction and therefore for the use in test systems directed to the detection of chlamydia-specific LPS
3. as a starting material for the manufacture of affinity media for the preparative purification of polyclonal or monoclonal antibodies, such as by chemical coupling to glass, silica gel, agarose gel, polyacrylamide gel and dextran gel via chemical bonds which can be obtained making use of the amino or carboxylic groups of the tetrasaccharide derivatives of formulae (I) and (II) and of the residue R in the tetrasaccharide derivatives of formula (I) of the present invention;
4. as immunogens for the manufacture of polyclonal or monoclonal antibodies;
5. as active vaccines;
6. as reagents for the characterization of the epitope specificity of polyclonal or monoclonal antibodies;
7. as therapeutic agents for the blocking of LPS specific receptor structures on host cells; and
8. as ligands for covalent coupling to different types of carriers, such as proteins, specifically enzymes, antibodies, avidine and specifically adjuvants, radioactive isotopes, fluorochromes, metal particles, spin-lables, dye stuffs, biotine and other markers used for detection, which can be used in the application referred to under items 1 to 7.

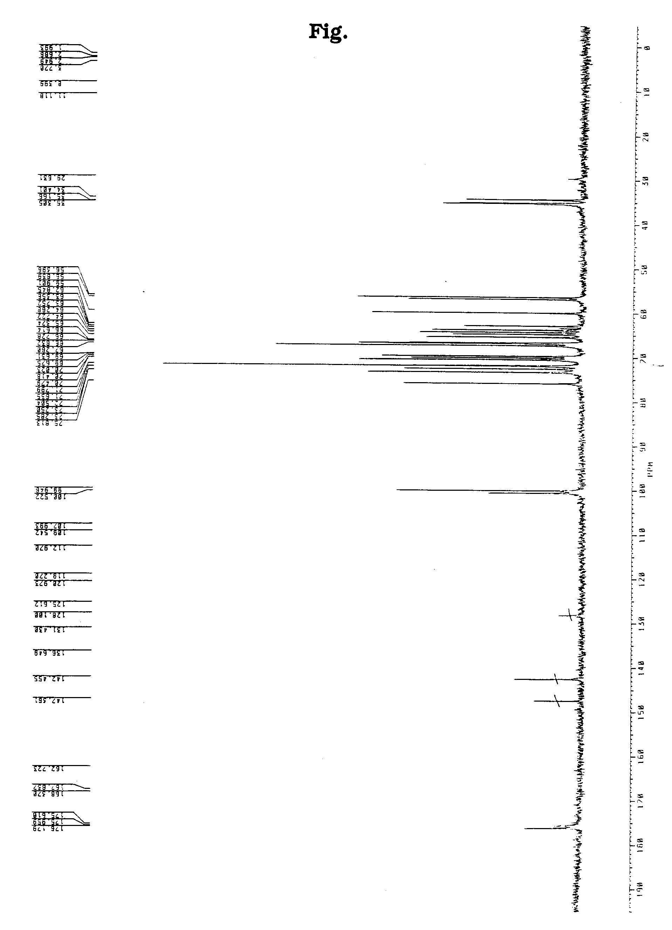

A more complete understanding of the invention is provided by reference to the following example and the drawing referred to therein.

SHORT DESCRIPTION OF THE DRAWING

The **single Fig.** shows the $^{13}C$ nuclear magnetic resonance spectrum of the tetrasaccharide derivative of the above formula (II) of the present invention.

The following examples demonstrate the manufacture of the tetrasaccharide derivatives of the invention.

**EXAMPLE 1**

**(a) Methyl-(7,8-O-carbonyl-3-deoxy-α-D-manno-2-octulopyranosyl)-onate-(2 → 6)-allyl-2-acetamido-2-deoxy-3,4-O-(tetraisopropyl-disiloxane-1,3-diyl)-β-D-glucopyranoside:**

A solution of 800 mg methyl-(4,5,7,8-tetra-O-acetyl-α-D-manno-2-octulopyranoside)-onate-bromide in 5 ml acetonitrile is added to a suspension of 500 mg fed for 20 h at room temperature. Thereafter the material is filtrated over a filter (Celite®), dissolved in dichloromethane and separated by shaking with an aqueous potassium iodide solution. This solution is dried over magnesium sulfate. thereafter the solvent is evaporated. The residue is purified on silica gel with a toluene/ethylacetate-mixture (1/1). To the sirup obtained (770 mg) which is dissolved in 20 ml methanol, 5 ml of a 0.1 molar methanolic sodium-methanolate solution is added and the material is agitated for 2.5 h. After the neutralization with an ion exchange resin (Dowex 50 ($H^+$)) the resin is filtrated and the filtrate is evaporated. The residue is dissolved in pyridine and 0.11 ml bisphosgen is added at - 20 °C. After agitating over night the material is evaporated and purified with silica gel making use of ethylacetate. This way the title compound is obtained. m. p. : 122-124 °C, $[\alpha]^{20}D$ + 50 ° (c = 1.0 in chloroform).

| Elementary analysis: | | | |
|---|---|---|---|
| Calculated: | C: 51.88; | H: 7.40; | N: 1.83. |
| Found: | C: 51.69; | H: 7.38; | N: 2.09. |

**(b) Methyl-(4,5,7,8-tetra-O-acetyl-3-deoxy-α-D-manno-2-octulopyranosyl)-onate-(2 → 8)-methyl-(4,5,7-tri-O-acetyl-3-deoxy-α-D-manno-2-octulopyranosyl)-onate-(2 → 4)-methyl-(7,8-O-carbonyl-3-deoxy-α-D-manno-2-octulopyranosyl)-onate-(2 → 6)-allyl-2-acetamido-2-deoxy-3,4-O-(tetraisopropyl-disiloxane-1,3-diyl)-β-D-glucopyranoside):**

A Solution of 203 mg methyl-(4,5,7,8-tetra-O-acetyl-3-deoxy-α-D-manno-2-octulopyranosyl)-onate-(2 → 8)-methyl-(4,5,7-tri-O-acetyl-3-deoxy-α-D-manno-2-octulopyranosyl)-onate-bromide in 5 ml acetonitrile is added to a suspension of 40 mg mercury cyanide, 57 mg mercury bromide, 140 mg methyl-(7,8-O-carbonyl-3-deoxy-α-D-manno-2-octulopyranosyl)-onate-(2 → 6)-allylacetamido-2-deoxy-3,4-O-(tetraisopropyldisiloxane-1,3-diyl)-β-D-glucopyranoside and 1 g of the molecular sieve 4Å and agitated for 48 h at room temperature. After diluting with ethyl acetate the material is filtrated over a filter (Celite®) extracted with a potassium iodide-solution, dried over magnesium sulfate and evaporated. After chromatography on silica gel with a toluene/ethylacetate-mixture (1/3) the title compound is obtained as a sirup. $[\alpha]^{20}D$ + 68 ° (c = 0.8 in chloroform).

**(c) Sodium-(3-deoxy-α-D-manno-2-octulopyranosyl)-onate-(2 → 8)-sodium-(3-manno-2-octulopyranosyl)-onate-(2 → 6)-allyl-2-acetamido-2-deoxy-β-D-glucopyranoside.**

A solution of 21.4 mg methyl-(4,5,7,8-tetra-O-acetyl-3-deoxy-α-D-manno-2-octulopyranosyl)-onate-(2 → 8)-methyl-(4,5,7-tri-O-acetyl-3-deoxy-α-D-manno-2-octulopyranosyl)-onate-(2 → 4)-methyl-(7,8-O-carbonyl-3-deoxy-α-D-manno-2-octulopyranosyl)-onate-(2 → 6)-allyl-2-acetamido-2-deoxy-3,4-O-(tetraisopropyldisiloxane-1,3-diyl)-β-D-glucopyranoside in 5 ml tetrahydrofurane is agitated with 30 mg tetrabutyl ammonium fluoride for 45 min at room temperature. The solution is neutralized and evaporated. The residue is dissolved in 10 ml methanol and 0.5 ml of a 0.1 molar methanolic sodium-methanolate solution is added. After agitating for 2 h, the residue is neutralized with an ion change resin (Dowex 50 ($H^+$)-), filtrated and evaporated. The residue is dissolved in water and 1 ml of 0.2 M sodium hydroxide is added. After agitating for 3 h, the material is brought to pH 8 with an ion exchange resin (Dowex 50 ($H^+$)), filtrated, purified over Biogel P-2 and lyophilyzed whereby the title compound is obtained as an amorphous powder. $[\alpha]^{20}_D$ + 49 ° (c = 0.7 in water).

**EXAMPLE 2**

According to a method known per se the plasmid pFEN 207 is introduced in a RE-chemotype of a gram-negative bacterium, which is hereinafter designated as recombinant RE-bacteria. These recombinant RE-bacteriae are cultured and multiplied by methods known per se. Thereafter the lipopolysaccharide

contained therein is obtained by methods known per se as well and is purified making use of well-known techniques. The lipopolysaccharide obtained is designated in the following as rRE-LPS.

a) MILD TREATMENT WITH HYDRAZINE:

The obtained rRE-LPS is dried in the vacuum with the aid of a drying agent. Thereafter the material is dissolved in anhydrous hydrazine (20mg/ml) and heated under nitrogen for 30 min under stirring to a temperature of 37 °C. Thereafter acetone is added to precipitate the product. The product is separated by centrifugation, is washed with acetone (two times), dried, dissolved in water (20mg/ml) precipitated again, centrifuged and washed as described. The drying provides de-O-acylated rRE-LPS having amide bonded 3-hydroxy fatty acids, i. e. the product rRE-LPS-$N_2H_4$-37.

b) TREATMENT WITH HYDROFLUORIC ACID FOR THE ELIMINATION OF THE PHOSPHATE RESIDUES:

The above material rRE-LPS-$N_2H_4$-37 is dried in vacuum making use of a drying agent. Thereafter the material is suspended in 48 % aqueous hydrofluoric acid and stirred at 0 °C for 48 h. Thereafter the product is precipitated by the addition of ethanol, is separated by centrifugation, washed with ethanol (two times), dried and dissolved in water (20mg/ml). The solution obtained is neutralized with a base (preferably NaOH or triethylamine) and lyophilized to provide the de-O-acylated and dephosphorylated product rRE-LPS-$N_2H_4$-37HF.

c) REDUCTION

The product obtained above, i. e. rRE-LPS-$N_2H_4$-37HF is dissolved in water (20mg/ml), treated with 20 mg sodium borohydride and left standing for 1 h at room temperature. Thereafter, acetic acid is added and the material is dialyzed against water. The drying by lyophilization provides the de-O-acylated, dephosphorylated and reduced product rRE-LPS-$N_2H_4$-37HFred.

This product is dried in vacuum making use of a drying agent. By adding anhydrous hydrazine (1 to 20 mg/ml) and heating to 85 °C under a nitrogen atmosphere for 7 days under stirring provides the tetrasaccharide derivative. By adding acetone the tetrasaccharide derivative is precipitated. The material is separated by centrifugation, washed with acetone (two times), dried and dissolved in water (50mg/ml). Thereafter the material is purified by preparative high voltage electrophoresis at a pH of 2.8 for 60 min at 50 V/cm. The elution of the fraction having a moving speed of 0.1 relatively to the 3-deoxy-D-manno-2-octulosonic acid provides the tetrasaccharide derivative. The $^{13}C$-NMR spectrum is shown in the Fig. and demonstrates the chemical structure shown in the above formula (II).

The NMR-spectrum has been recorded by making use of a solution of the tetrasaccharide derivative in deuterium oxide (6.3 mg in 500 $\mu$l) and measured in a Bruker instrument (model AM 360L) at 90.56 MHz at 296 °K. $CH_3CN$ was used as the internal standard (1.700 ppm).

The obtained tetrasaccharide derivatives of the present invention and specifically the derivative of formula (II) have epitope specificity for chlamydiae which has been shown immunochemically by means of the inhibition value obtained with monoclonal antibodies. The inhibition values were determined by the passive hemolysis inhibition assay in the following way: sheep erythrocytes were passively coated with chlamydial-LPS which were lysed by the respective monoclonal antibody in the presence of guinea-pig serum as a source of complement. The monoclonal anitbodies were diluted to yield 2-3 hemolytic units in 25 $\mu$l of veronal-buffered saline. This amount of antibody was preincubated with various amounts of inhibitor and the amount causing 50 % inhibition of lysis was determined. The test was carried out in 96-well microtiter plates.

As a comparative material the allyl glycoside of the trisaccharide $\alpha$-KDOp-2.8-$\alpha$-KDOp-2.4-$\alpha$-KDOp- was copolymerized with acrylamide by a method known per se. The resulting product was adjusted to 1 mg/ml; it contained 37 nmol of ligand per mg of copolymerisate (KDO3-PA). The manufacture of these trisaccharides has been described by P. Kosma, G. Schulz, and H. Brade (Carbohydr. Res. 183:183-188 (1988)).

The tetrasaccharide derivative of the invention was coupled to bovine serum albumin (BSA) which had been preactivated with glutardialdehyde. The resulting product was reduced with sodium borohydride in water, dialysed and adjusted to 1 mg/ml with regard to BSA; it contained 32 nmol of ligand per mg of conjugate.

The results obtained in this inhibition test are summarized in the following table 1:

Table 1

| Inhibitor | 50% inhibition value (pmol/ml) obtained with monoclonal antibody: | | | |
|---|---|---|---|---|
| | S5-10 | S10-3 | S15-2 | S15-6 |
| $KDO_3$-PA | 275 | 138 | 2.2 | 4.4 |
| Tetrasaccharide-BSA | 8 | 2 | 2 | 2 |

The monoclonal antibodies used in the above inhibition test have been obtained as follows:
Monoclonal antibodies: Murine monoclonal antibodies were obtained by a conventional protocol known per se after immunizing mice with purified elementary bodies of C. psittaci (clone S5-10 (IgG3) and S10-3 (IgM)) or with rRE LPS incorporated into liposomes (S15-2 and S15-6, both IgM). Hybridomas were screened for the production of chlamydia-specific antibodies using an enzyme-linked immunosorbent assay with rRE LPS as the solid phase. Positive clones were selected, grown up and tested with RE LPS from Salmonella minnesota and Escherichia coli. Only those clones which were positive with rRE LPS but negative with RE LPS from Salmonella minnesota and Escherichia coli were considered chlamydia-specific.

The tetrasaccharide derivatives of the invention comprise at least one amino group which allows a covalent coupling to different types of carriers, such as proteins in general, enzymes, antibodies and avidine, adjuvants, radioactive isotopes, fluorochromes, metal particles, polystyrene particles,. spin-labels, dyestuffs, biotine or other markes for the detection of chlamydia-specific antibodies and antigens, such as derivatized glass, silica gel, agarose gel, polyacrylamide gel or dextrane gel or other substances which may be used for immobilizing the tetrasaccharide derivatives.

The following examples demonstrate the use of the tetrasaccharide derivatives of the invention.

**EXAMPLE 3**

The tetrasaccharide derivative is reacted with an excess of glutardialdehyde to provide a Schiff base in such a way that a reactive carbonyl group of the glutardialdehyde remains. The product is separated from free glutardialdehyde, free tetrasaccharide derivative and polymers of the tetrasaccharide derivative and glutardialdehyde by means of gel-permeation chromatography. The product can be reacted with the amino groups of a carrier material by providing a further Schiff base. The reduction of the Schiff base with sodium borohydride provides a stable bond between the tetrasaccharide derivative and the carrier. The reduction of this Schiff base with radioactive sodium borohydride yields a stable bond between the tetrasaccharide derivative and the carrier with the introduction of a radioactive isotope (tritium).

**EXAMPLE 4**

The tetrasaccharide derivative can be reacted with the carbonyl groups of carriers which are obtained by periodate oxidation of the saccharide part of glycosylated proteins (for example antibodies or enzymes) which again provides a Schiff base. The reduction of the Schiff base with sodium borohydride provides a stable bond between the tetrasaccharide derivative and the carrier. The reduction of the Schiff base with radioactive sodium borohydride yields a stable bond between the tetrasaccharide derivative and the carrier with the introduction of a radioactive isotope (tritium).

**EXAMPLE 5**

A carrier having primary amino groups is reacted with an excess of glutardialdehyde to provide a Schiff base where the reaction is carried out in such a way that a reactive carbonyl group of the glutardialdehyde remains. The product is separated from free glutardialdehyde, free carrier and polymers of the carrier and the glutardialdehyde by means of gel-permeation chromatography or centrifugation in case of insoluble carriers. The product can be reacted with the amino groups of the tetrasaccharide derivative producing a Schiff base. The reduction of the Schiff base with sodium borohydride provides a stable bond between the tetrasaccharide derivative and the carrier. The reduction of the Schiff base with radioactive sodium borohydride yields a stable bond between the oligosaccharide and the carrier with the introduction of a radioactive isotope (tritium).

**EXAMPLE 6**

Coupling of the tetrasaccharide derivatives to a protein

The tetrasaccharide derivatives are coupled according to examples 2 to 4 to bovine serum albumine (BSA) providing a reaction product which is designated as tetrasaccharide-BSA. The chemical analysis of the tetrasaccharide-BSA (determination of KDO and the glucosamine content) has demonstrated that between 1 and 100 nmol tetrasaccharide derivatives per mg tetrasaccharide-BSA were coupled. This material has been checked for its chlamydia-specificity by the inhibition of the passive hemolysis making use of chlamydia-specific monoclonal antibodies. The material showed the same activity as authentic chlamydia-LPS or rRE LPS.

**EXAMPLE 7**

Coupling of the tetrasaccharide derivatives to a derivatized gel

The tetrasaccharide derivative was coupled according to the method of examples 2 to 4 above to aminohexyl sepharose 4B. The reaction product is designated in the following as tetrasaccharide-AH-sepharose 4B. Chlamydia-specific monoclonal antibodies bind to tetrasaccharide-AH-sepharose 4B and can be eluted with a glycine/HCl buffer at a low pH. The results obtained in this test are shown in the following table 2:

Table 2

| Chlamydia-specific monoclonal antibodies | Volume (ml) | antibody titer in the passive hemolysis test with erythrocytes that have been loaded with Chlamydia LPS |
|---|---|---|
| starting material | 5 | 128 |
| non-bound part | 5 | <4 |
| eluted part | 1 | 512 |

## Claims

**1.** The tetrasaccharide derivatives of the general formula (I)

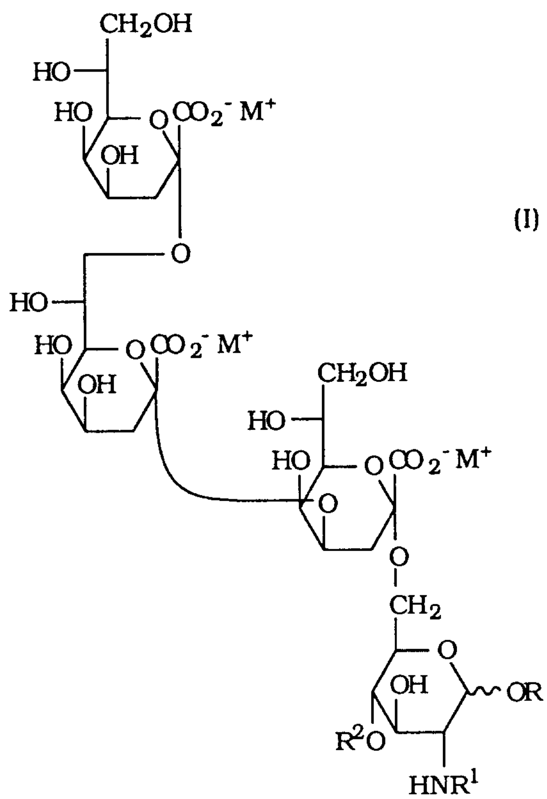

(I)

wherein
R is hydrogen, alkyl, alkenyl, aminoalkyl or the residue of an aldose, a ketose or a sugar alcohol carrying phosphate, sulfate, amino or carboxyl groups and having up to 4 to 8 carbon atoms,
$R^1$ is hydrogen or an acyl group,
$R^2$ is hydrogen or $PO(OH)_2$ and
$M^+$ is a neutralizing cation.

**2.** The tetrasaccharide derivative 3-deoxy-α-D-manno-2-octulopyranosyl-onate-(2 → 8)-3-deoxy-α-D-manno-2-octulopyranosyl-onate-(2 → 4)-3-deoxy-α-D-manno-2-octulopyranosyl-onate-(2 → 6)-2-amino-2-deoxy-β-D-glucopyranosyl-(1 → 6)-2-amino-2-deoxy-D-glucitol acording to claim 1 having the following formula (II)

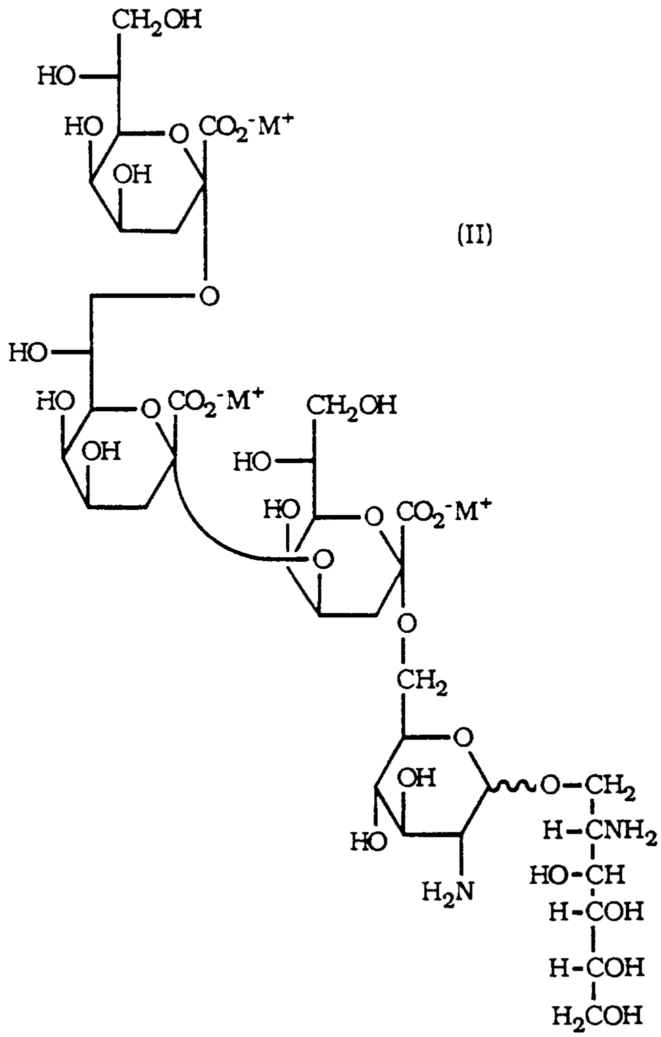

(II)

wherein $M^+$ is a neutralizing cation.

3. The tetrasaccharide derivatives according to claims 1 and 2, **characterized in that** $M^+$ is a sodium, potassium, calcium, ammonium or substituted ammonium cation.

4. A process for the manufacture of the tetrasaccharide derivatives according to claims 1 to 3, **characterized by** reacting a disaccharide of the general formula (III)

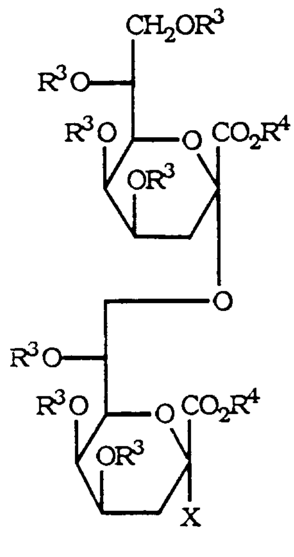

(III)

(III)

wherein $R^3$ are protecting groups
$R^4$ are alkyl groups and
X is halogen,
by a method known per se with a disaccharide of the general formula (IV) or a trisaccharide of the general formula (V)

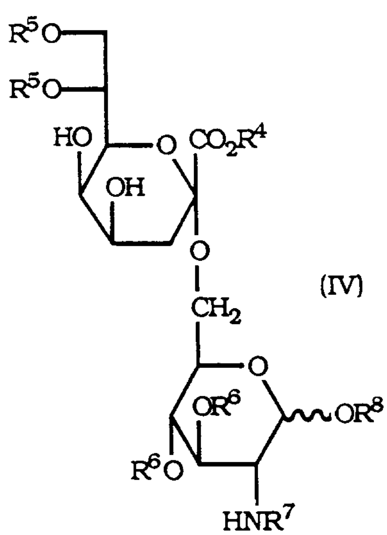

(IV)

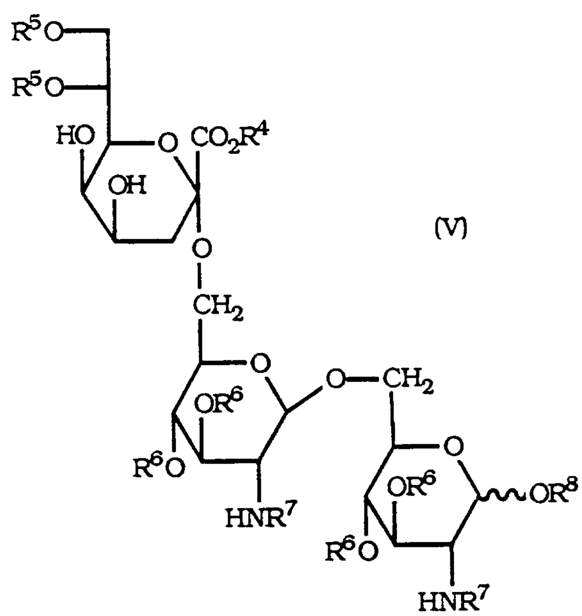

(V)

wherein $R^4$ is as defined above
$R^5$ and $R^6$ independently are bifunctional protecting groups, such as cyclic acetal or ketal groups, ester functions or silyl ether groups,
$R^7$ independently are acyl groups having 2 to 24 carbon atoms or 3-hydroxyacyl groups having 4 to 24 carbon atoms and
$R^8$ are alkyl groups having 1 to 4 carbon atoms or alkenyl groups having 3 to 8 carbon atoms,
in an inert solvent and in the presence of a heavy metal salt catalyst, chromatographically separating the tetrasaccharide derivatives and thereafter eliminating the protecting groups by a method known per se.

5. A process for the manufacture of the tetrasaccharide derivatives according to claims 1 to 3, **characterized by**

a) transforming the plasmide pFEN207 in a RE-chemotype of a gram-negative bacterium (hereinafter designated as recombinant RE-bacterieae - rRE),
b) culturing said recombinant RE-bacteria by a method known per se,
c) isolating and purifying the lipopolysaccharide (rRe-LPS) of the multiplied recombinant RE-bacteria by a method known per se,
d) dissolving rRE-LPS in anhydrous hydrazine, stirring under a nitrogen atmosphere at a temperature of 37 ° C, purifying and drying the product (rRE-LPS-$N_2H_4$-37),
e) suspending and stirring rRE-LPS-$N_2H_4$-37 at a temperature of 0 ° C in aqueous HF, purifying, neutralizing and drying the product (rRE-LPS-$N_2H_4$-37-HF),
f) dissolving rRE-LPS-$N_2H_4$-37-HF in water reducing at room temperature, dialyzing and drying the product (rRE-LPS-$N_2H_4$-37-H-red),
g) adding hydrazine to rRE-LPS-$N_2H_4$-37-HF-red, stirring under a nitrogen atmosphere at an elevated temperature, purifying, drying and isolating the tetrasaccharide derivatives obtained by preparative high voltage electrophoresis, and
h) introducing the desired substituents R by methods known per se.

**6.** The process according to claim 5, **characterized by**
dissolving rRE-LPS in step d) in anhydrous hydrazine (1 to 20mg/ml), stirring for 30 min under a nitrogen atmosphere at a temperature of 37 ° C, precipitating by adding acetone, separating, purifying and drying the product (rRE-LPS-$N_2H_4$-37),
suspending and stirring rRE-LPS-$N_2H_4$-37 in step e) for 48 h at a temperature of 0 ° C in 48% aqueous HF, precipitating by adding ethanol, separating, purifying, drying, dissolving the product in water (1 to 20mg/ml) neutralizing with NaOH or triethylamine and lyophilizing the product (rRE-LPS-$N_2H_4$-37-HF),
dissolving rRE-LPS-$N_2H_4$-37-HF in step f) in water (1 to 20mg/ml) adding sodium borohydride and reducing at room temperature for 15 min to 2 h adding acetic acid. dialyzing against water and lyophilizing the product (rRE-LPS-$N_2H_4$-37-HF-red), and
adding in step g) (0,1 to 2 ml/20 mg) anhydrous hydrazine to rRE-LPS-$N_2H_4$-37-HF-red dried in vacuum with a drying agent, stirring for 5 to 14 days under a nitrogen atmosphere at a temperature of 85 to 100 ° C, precipitating by adding acetone, separating, purifying drying, dissolving in water(5 to 100 mg/ml) and isolating the tetrasaccharide derivative by preparative high voltage electrophoresis at pH 2.8 and 50V/cm.

**7.** The use of the tetrasaccharide derivatives according to claims 1 or 2 for the manufacture of reagents and compositions for the diagnosis and therapy of human and animal chlamydial infections.

**8.** The use according to claim 7, **characterized by** using the tetrasaccharide derivatives as an antigen for the direct assay of lipopolysaccharide antibodies against Chlamydiae, as an inhibitor of the chlamydia specific antigen antibody reaction, as an immunogen for the manufacture of monoclonal or polyclonal antibodies, as an active agent for the manufacture of affinity media for preparative purification of monoclonal or polyclonal antibodies, as an active vaccine, as a reagent for the characterization of the epitope specificity of monoclonal or polyclonal antibodies, as a therapeutic agent for the blocking of lipopolysaccharide recognizing receptor structures of Chlamydiae on host cells or as a ligand for coupling with carrier materials.

**Patentansprüche**

1. Tetrasaccharidderivate der allgemeinen Formel (I)

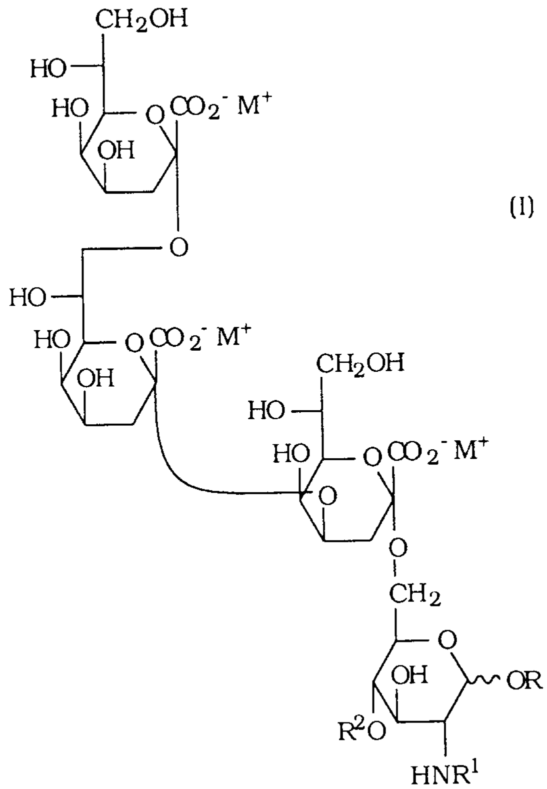

(I)

worin bedeuten:
R Wasserstoff, Alkyl, Alkenyl, Aminoalkyl oder den Rest einer Aldose, einer Ketose oder eines Zuckeralkohols, welcher Phosphat-, Sulfat-, Amino- oder Carboxylgruppen trägt und bis zu 4 bis 8 Kohlenstoffatome aufweist,
$R^1$ Wasserstoff oder eine Acylgruppe,
$R^2$ Wasserstoff oder $PO(OH)_2$ und
$M^+$ ein neutralisierendes Kation.

2. Das Tetrasaccharidderivat 3-Desoxy-α-D-manno-2-octulopyranosyl-onat-(2 → 8)-3-desoxy-α-D-manno-2-octulopyranosyl-onat-(2 → 4)-3-desoxy-α-D-manno-2-octulopyranosyl-onat-(2 → 6)-2-amino-2-desoxy-β-D-glucopyranosyl-(1 → 6)-2-amino-2-desoxy-D-glucitol nach Anspruch 1 der folgenden Formel (II)

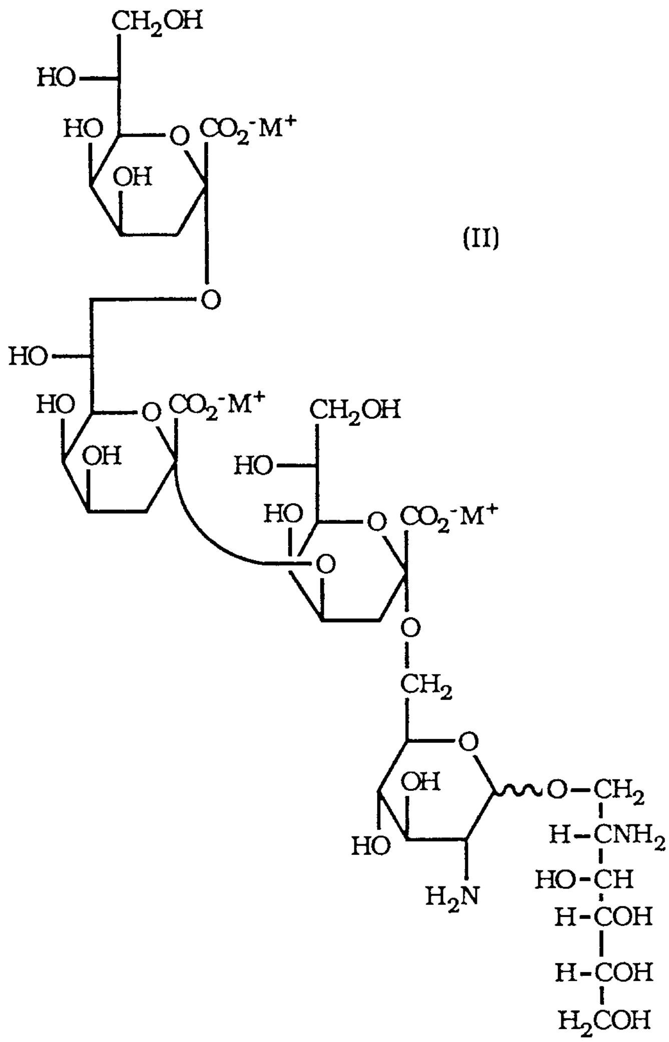

(II)

worin $M^+$ ein neutralisierendes Kation ist.

3. Tetrasaccharidderivate nach den Ansprüchen 1 und 2, **dadurch gekennzeichnet**, daß $M^+$ ein Natrium-, Kalium-, Calcium-, Ammonium- oder substituiertes Ammoniumkation ist.

4. Verfahren zur Herstellung der Tetrasaccharidderivate nach den Ansprüchen 1 bis 3, **gekennzeichnet durch** die Umsetzung eines Disaccharids der allgemeinen Formel (III),

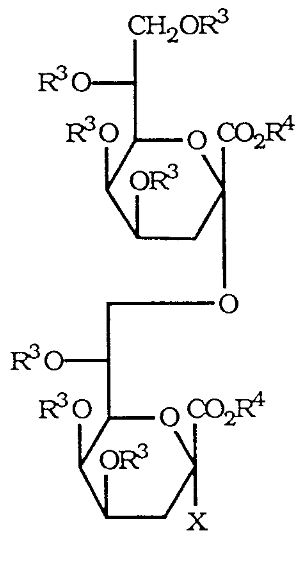

(III)

worin $R^3$ Schutzgruppen sind,

$R^4$ Alkylgruppen sind und

X Halogen ist,

durch ein an sich bekanntes Verfahren mit einem Disaccharid der allgemeinen Formel (IV) oder einem Trisaccharid der allgemeinen Formel (V),

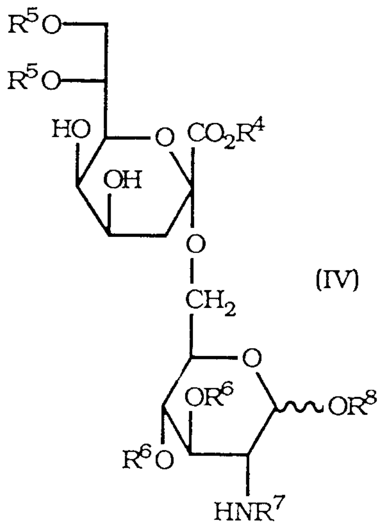

(IV)

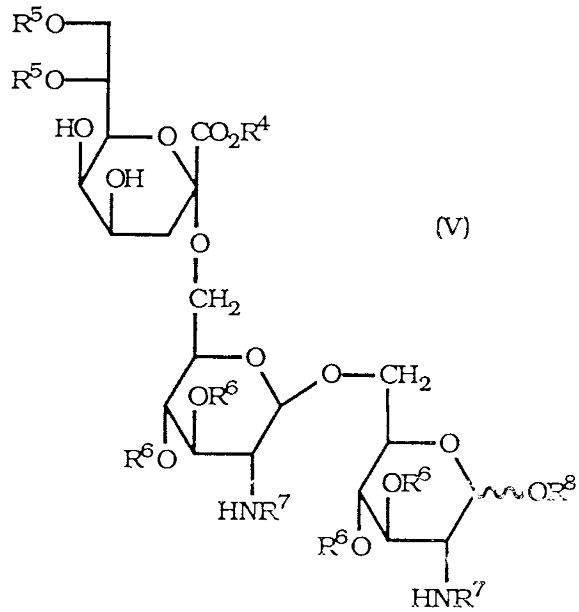

(V)

worin $R^4$ wie oben definiert ist,

$R^5$ und $R^6$ unabhängig voneinander bifunktionelle Schutzgruppen sind, wie etwa cyclische Acetal- oder Ketalgruppen, Esterfunktionen oder Silylethergruppen,

$R^7$ unabhängig voneinander Acylgruppen mit 2 bis 24 Kohlenstoffatomen oder 3-Hydroxyacylgruppen mit 4 bis 24 Kohlenstoffatomen sind, und

$R^8$ Alkylgruppen mit 1 bis 4 Kohlenstoffatomen oder Alkenylgruppen mit 3 bis 8 Kohlenstoffatomen sind,

in einem inerten Lösungsmittel und in Gegenwart eines Schwermetallsalz-Katalysators, chromatografische Abtrennung der Tretrasaccharidderivate und danach Eliminierung der Schutzgruppen durch ein an sich bekanntes Verfahren.

5. Verfahren zur Herstellung der Tetrasaccharidderivate nach den Ansprüchen 1 bis 3, **gekennzeichnet durch**
   a) Transformieren des Plasmids pFEN207 in einen RE-Chemotyp eines gramnegativen Bakteriums (nachfolgend als rekombinante RE-Bakterien-rRE bezeichnet),
   b) Kultivieren der rekombinanten RE-Bakterien durch ein an sich bekanntes Verfahren,
   c) Isolieren und Reinigen des Lipopolysaccharids (rRe-LPS) dervervielfachten rekombinanten RE-Bakterien durch ein an sich bekanntes Verfahren,
   d) Auflösen von rRE-LPS in einem wasserfreien Hydrazin, Rühren unter einer Stickstoffatmosphäre bei einer Temperatur von 37 ° C, Reinigen und Trocknen des Produkts (rRE-LPS-$N_2H_4$-37),
   e) Suspendieren und Rühren von rRE-LPS-$N_2H_4$-37 bei einer Temperatur von 0 ° C in wäßriger HF, Reinigen, Neutralisieren und Trocknen des Produkts (rRE-LPS-$N_2H_4$-37-HF),
   f) Auflösen von rRE-LPS-$N_2H_4$-37-HF in Wasser, Reduzieren bei Raumtemperatur, Dialysieren und Trocknen des Produkts (rRE-LPS-$N_2H_4$-37-H-red),
   g) Zugeben von Hydrazin zu rRE-LPS-$N_2H_4$-37-HF-red, Rühren unter einer Stickstoffatmosphäre bei einer erhöhten Temperatur Reinigen, Trocknen und Isolieren der erhaltenen Tetrasaccharidderivate durch präparative Hochspannungs-Elektrophorese, und
   h) Einführen der erwünschten Substituenten R durch an sich bekannte Verfahren.

6. Verfahren nach Anspruch 5, **gekennzeichnet durch**
   Auflösen von rRE-LPS in Schritt d) in wasserfreiem Hydrazin (1 bis 20 mg/ml), Rühren während 30 Minuten unter einer Stickstoffatmosphäre bei einer Temperatur von 37 ° C, Ausfällen durch Zusetzen von Aceton, Abtrennen, Reinigen und Trocknen des Produkts (rRE-LPS-$N_2H_4$-37), Suspendieren und Rühren von rRE-LPS-$N_2H_4$-37 in Schritt e) während 48 Stunden bei einer Temperatur von 0 ° C in 48%-iger wäßriger HF, Ausfällen durch Zugeben von Ethanol, Abtrennen, Reinigen, Trocknen, Auflösen des Produkts in Wasser (1 bis 20 mg/ml), Neutralisieren mit NaOH oder Triethylamin und Lyophilisieren des Produkts (rRE-LPS-$N_2H_4$-37-HF),
   Auflösen von rRE-LPS-$N_2H_4$-37-HF in Schritt f) in Wasser (1 bis 20 mg/ml), Zugeben von Natriumborhydrid und Reduzieren bei Raumtemperaturwährend 15 Minuten bis 2 Stunden, Zugeben von Essigsäure, Dialysieren gegen Wasser und Lyophilisieren des Produkts (rRE-LPS-$N_2H_4$-37-HF-red), und
   Zugeben in Schritt g) (0,1 bis 2 ml/20mg) von wasserfreiem Hydrazin zu rRE-LPS-$N_2H_4$-37-HF-red, welches mit einem Trocknungsmittel im Vakuum getrocknet worden ist, Rühren während 5 bis 14 Tagen unter einer Stickstoffatmosphäre bei einer Temperatur von 85 bis 100 ° C, Ausfällen durch Zusetzen von Aceton, Abtrennen, Reinigen, Trocknen, Auflösen in Wasser (5 bis 100 mg/ml) und Isolieren des Tretrasaccharidderivats durch präparative Hochspannungs-Elektrophorese bei pH 2,8 und 50V/cm.

7. Verwendung der Tretrasaccharidderivate nach den Ansprüchen 1 oder 2 zur Herstellung von Reagenzien und Zusammensetzungen für die Diagnose und Therapie von Chlamydieninfektionen bei Mensch und Tier.

8. Verwendung nach Anspruch 7, **gekennzeichnet durch** die Verwendung der Tetrasaccharidderivate als Antigen für die direkte Analyse von Lipopolysaccharid-Antikörpern gegen Chlamydien, als ein Inhibitor der chlamydienspezifischen Antigen-Antikörper-Reaktion, als ein Immunogen für die Herstellung monoklonaler oder polyklonaler Antikörper, als Wirkstoff für die Herstellung von Affinitätsmedien für die präparative Reinigung monoklonaler oder polyklonaler Antikörper, als aktive Vakzine, als ein Reagens für die Charakterisierung der Epitopspezifität monoklonaler oder polyklonaler Antikörper, als therapeutisches Mittel für die Blockade Lipopolysaccharid-erkennender Rezeptor-Strukturen von Chlamydien auf Wirtszellen oder als ein Ligand zur Kopplung mit Trägermaterialien.

## Revendications

1. Dérivés de tétrasaccharide de formule générale (I)

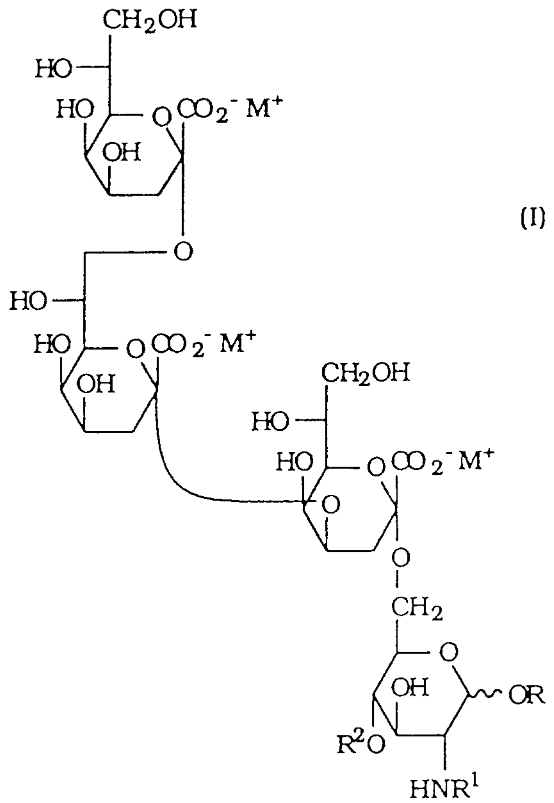

(I)

dans laquelle
R est un atome d'hydrogène, un groupe alkyle, un groupe alcényle, un groupe aminoalkyle ou le reste d'un aldose, d'un cétose ou d'un sucre-alcool portant des groupes phosphate, sulfate, amino ou carboxyle et ayant jusqu'à 4 à 8 atomes de carbone,
$R^1$ est un atome d'hydrogène ou un groupe acyle,
$R^2$ est un atome d'hydrogène ou $PO(OH)_2$ et
$M^+$ est un cation neutralisant.

2. Dérivé de tétrasaccharide 3-désoxy-α-D-manno-2-octulopyrannosylonate-(2→8)-3-désoxy-α-D-manno-2-octulopyrannosylonate-(2→4)-3-désoxy-α-D-manno-2-octulopyrannosylonate-(2→6)-2-amino-2-désoxy-β-D-glucopyrannosyl-(1→6)-2-amino-2-désoxy-D-glucitol selon la revendication 1, répondant à la formule (II) suivante

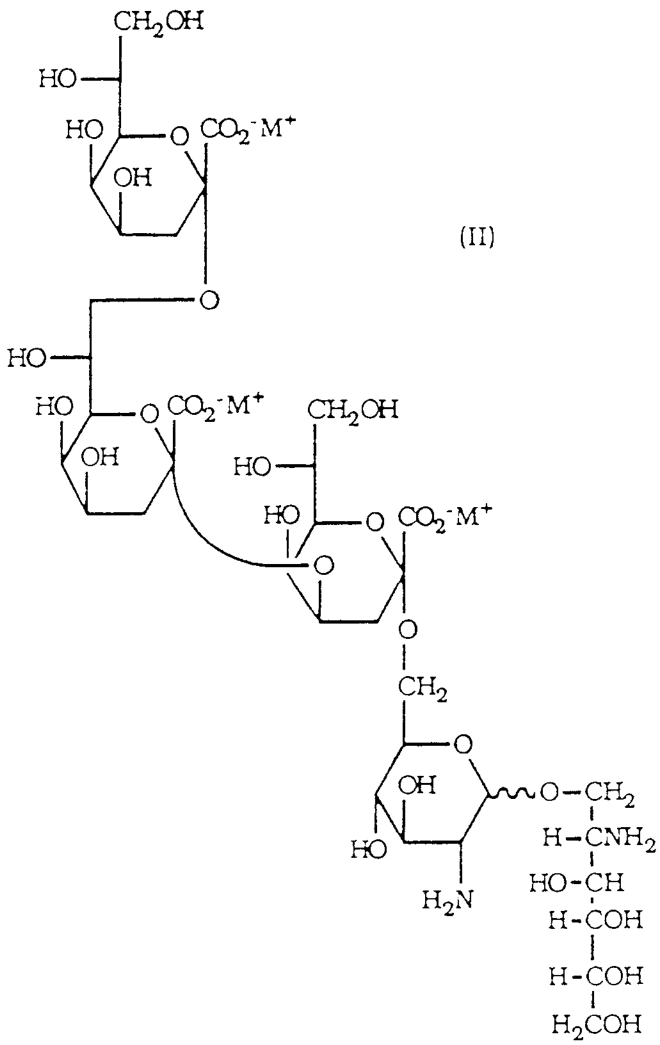

(II)

dans laquelle $M^+$ est un cation neutralisant.

**3.** Dérivés de tétrasaccharide selon les revendications 1 et 2, caractérisés en ce que $M^+$ est un cation sodium, potassium, calcium, ammonium ou ammonium substitué.

**4.** Procédé pour la préparation des dérivés de tétrasaccharide selon les revendications 1 à 3, caractérisé par la réaction d'un disaccharide de formule générale (III)

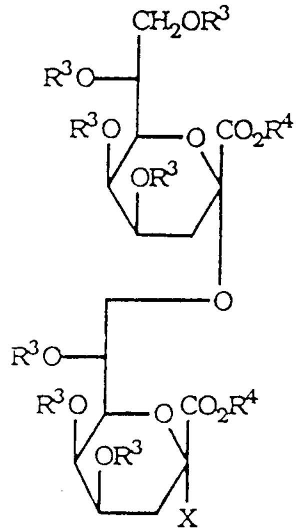

(III)

dans laquelle les $R^3$ sont des groupes protecteurs,

les $R^4$ sont des groupes alkyle et

X est un atome d'halogène,

selon un procédé connu en soi, avec un disaccharide de formule générale (IV) ou un trisaccharide de formule générale (V)

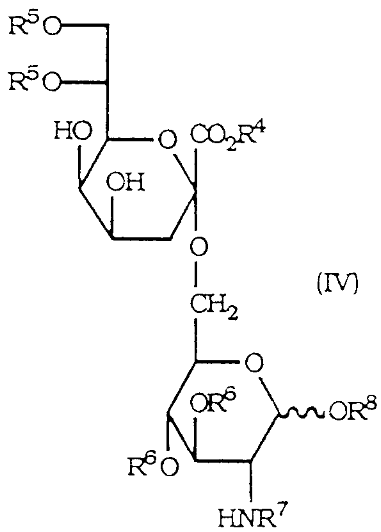

(IV)

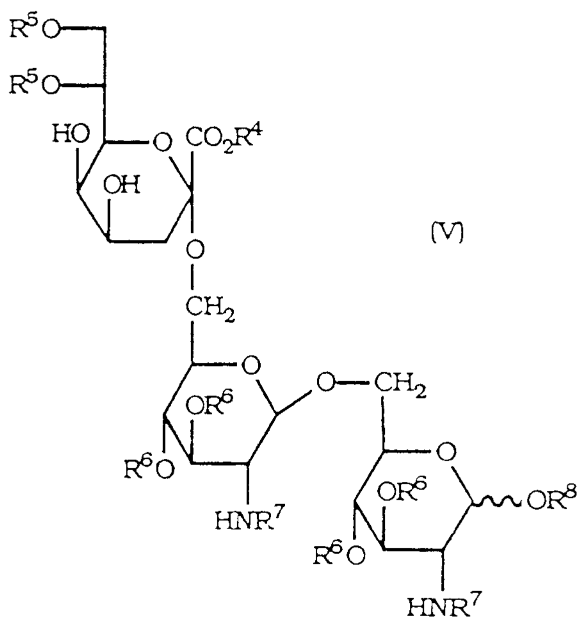

(V)

où $R^4$ est tel que défini ci-dessus,

$R^5$ et $R^6$, indépendamment, sont des groupes protecteurs bifonctionnels tels que des groupes acétal ou cétal cycliques, des fonctions ester ou des groupes éther silylique,

les $R^7$ sont indépendamment des groupes acyle ayant 2 à 24 atomes de carbone ou des groupes 3-hydroxyacyle ayant 4 à 24 atomes de carbone et

les $R^8$ sont des groupes alkyle ayant 1 à 4 atomes de carbone ou des groupes alcényle ayant 3 à 8 atomes de carbone, dans un solvant inerte et en présence d'un catalyseur à sel de métal lourd, la séparation chromatographique des dérivés de tétrasaccharide puis l'élimination des groupes protecteurs selon un procédé connu en soi.

**5.** Procédé pour la préparation des dérivés de tétrasaccharide selon les revendications 1 à 3, caractérisé en ce qu'il consiste à

a) transformer avec le plasmide pFEN207 le chimiotype RE d'une bactérie Gram négatif (appelée ci-après bactérie-RE-rRE),

b) cultiver ladite bactérie-RE recombinée selon un procédé connu en soi,

c) isoler et purifier le lipopolysaccharide (rRE-LPS) de la bactérie-RE recombinée multipliée, selon un procédé connu en soi,

d) dissoudre le rRE-LPS dans de l'hydrazine anhydre, agiter sous une atmosphère d'azote à une température de 37 °C, purifier et sécher le produit (rRE-LPS-$N_2H_4$-37),

e) mettre en suspension et agiter le rRE-LPS-$N_2H_4$-37 à une température de 0 °C dans de l'HF aqueux, purifier, neutraliser et sécher le produit (rRE-LPS-$N_2H_4$-37-HF),

f) dissoudre le rRE-LPS-$N_2H_4$-37-HF dans de l'eau, réduire à la température ordinaire, dialyser et sécher le produit (rRE-LPS-$N_2H_4$-37-HF-red),

g) ajouter de l'hydrazine au rRE-LPS-$N_2H_4$-37-HF-red, agiter sous une atmosphère d'azote à une température élevée, purifier, sécher et isoler les dérivés de tétrasaccharide obtenus par électrophorèse préparative à haute tension et

h) introduire les substituants R désirés selon des procédés connus en soi.

**6.** Procédé selon la revendication 5, caractérisé par :
la dissolution du rRE-LPS dans l'étape d) dans de l'hydrazine anhydre (1 à 20 mg/ml), l'agitation pendant 30 minutes sous une atmosphère d'azote à une température de 37 °C, la précipitation par addition d'acétone, la séparation, la purification et le séchage du produit (rRE-LPS-$N_2H_4$-37),
la mise en suspension et l'agitation du rRE-LPS-$N_2H_4$-37 dans l'étape e), pendant 48 heures à une température de 0 °C, dans de l'HF aqueux à 48 %, la précipitation par addition d'éthanol, la séparation, la purification, le séchage, la dissolution du produit dans l'eau (1 à 20 mg/ml), la neutralisation avec NaOH ou la triéthylamine, et la lyophilisation du produit (rRE-LPS-$N_2H_4$-37-HF),
la dissolution du rRE-LPS-$N_2H_4$-37-HF dans l'étape f) dans de l'eau (1 à 20 mg/ml), l'addition de borohydrure de sodium et la réduction à la température ordinaire pendant 15 minutes à 2 heures, l'addition d'acide acétique, la dialyse contre de l'eau et la lyophilisation du produit (rRE-LPS-$N_2H_4$-37-HF-red), et
l'addition dans l'étape g) d'hydrazine anhydre (0,1 à 2 ml/20 mg) au rRE-LPS-$N_2H_4$-37-HF-red séché sous vide avec un agent déshydratant, l'agitation pendant 5 à 14 jours sous une atmosphère d'azote à une température de 85 à 100 °C, la précipitation par addition d'acétone, la séparation, la purification, le séchage, la dissolution dans l'eau (5 à 100mg/ml) et l'isolement du dérivé de tétrasaccharide par électrophorèse préparative à haute tension à un pH de 2,8 et à 50 V/cm.

**7.** Utilisation des dérivés de tétrasaccharide selon les revendications 1 ou 2 pour la préparation de réactifs et de compositions pour le diagnostic et le traitement des infections à chlamydia de l'homme et des animaux.

**8.** Utilisation selon la revendication 7, caractérisée par l'utilisation des dérivés de tétrasaccharide en tant qu'antigène pour l'essai direct d'anticorps lipopolysaccharidiques contre les chlamydia, en tant qu'inhibiteur de la réaction antigène-anticorps spécifique des chlamydia, en tant qu'immunogène pour la préparation d'anticorps monoclonaux ou polyclonaux, en tant qu'agent actif pour la préparation de milieu d'affinité pour la purification préparative d'anticorps monoclonaux ou polyclonaux, en tant que vaccin actif, en tant que réactif pour la caractérisation de la spécificité d'épitope d'anticorps monoclonaux ou polyclonaux, en tant qu'agent thérapeutique pour le blocage des structures réceptrices reconnaissant les lipopolysaccharides de chlamydia sur des cellules hôtes ou en tant que ligand pour le couplage avec des matières supports.

Fig.